# EUROPEAN PATENT SPECIFICATION

(11) **EP 1 608 752 B1**
(45) Date of publication and mention of the grant of the patent: **16.12.2009**
(21) Application number: 04758687.0
(22) Date of filing: 01.04.2004
(51) Int. Cl.: C12N 15/10

(54) **METHOD FOR ISOLATING NUCLEIC ACIDS**
METHODE ZUR ISOLATION VON NUKLEINSÄUREN
PROCEDE D'ISOLEMENT D'ACIDES NUCLEIQUES

(30) Priority: 02.04.2003 US 406141
(43) Date of publication of application: 28.12.2005
(73) Proprietor: Agencourt Bioscience Corporation, Beverly, MA 01915 (US)
(72) Inventor: MCKERNAN, Kevin, Marblehead, MA 01945 (US); ZIAUDDIN, Junaid, Sommerville, MA 02143-3618 (US)
(74) Representative: Lock, Graham James
(86) International application number: PCT/US2004/009960
(87) International publication number: WO 2004/090132

(56) References cited:
- WO-A-99/58664
- WO-A-02/055727
- US-A1- 2003 235 839
- HAWKINS T L ET AL: "DNA PURIFICATION AND ISOLATION USING A SOLID-PHASE" NUCLEIC ACIDS RESEARCH, OXFORD UNIVERSITY PRESS, SURREY, GB, vol. 22, no. 21, 1994, pages 4543-4544, XP000602048 ISSN: 0305-1048

## Description

### FIELD OF THE INVENTION

The present invention relates to a method for isolating nucleic acids.

### BACKGROUND OF THE INVENTION

Many molecular biology applications, such as capillary electrophoresis, nucleotide sequencing, reverse transcription cloning and gene therapy protocols, which contemplate the transfection, transduction or microinjection of mammalian cells, require the isolation of high quality nucleic acid preparations.

The advent of demandimg molecular biology applications has increased the need for high-throughput, and preferably readily automatable, purification protocols capable of producing high quality nucleic acid preparations. Although recent technological advancements and the advent of robotics have facilitated the automation of sequencing reactions and gel reading steps, throughput is still limited by the availability of readily automatable methods of nucleic acid purification.

### SUMMARY OF THE INVENTION

As described herein, Applicants provide a method in which genomic nucleic acid of a cell can be separated from nucleic acid having a molecular weight that is lower than the molecular weight of the genomic nucleic acid (e.g., plasmid DNA) of the cell, directly from a cell growth culture. In addition, Applicants provide a method in which genomic nucleic acid can be separated from nucleic acid having a molecular weight that is lower than the molecular weight of the genomic nucleic acid in a cell lysate, without the need to prepare a cleared lysate.

Traditional alkaline lysis requires the following steps: concentrating or pelleting cells diluted in growth media; centrifuging or vortexing; lysing cells with alkaline detergent; shaking and/or agitating lysate; adding neutralization buffer; filtering and/or manipulating sample to remove the flocculent mass; adding a solid phase carrier; and adding binding buffer. Additional purification steps are generally required to remove the detergents and salts as follows: addition of solid phase carriers and addition of a binding buffer.

WO 02/055727 discloses size-dependent DNA isolation using COOH coated magnetic beads by selecting an appropriate concentration of the precipitating reagent (polyalkylene glycol and salt).

The isolation of plasmid DNA as exemplified consists of two steps: first removing the genomic DNA with magnetic beads and then binding the plasmid DNA in the remaining solution to new beads.

An advantage of the invention is that it allows for a simplified procedure for separating genomic nucleic acid of a cell from nucleic acids having a molecular weight lower than the molecular weight of the genomic nucleic acid of the cell. By providing solid phase carriers and a reagent that causes lysis of cells and precipitation of the nucleic acid of the cells onto the solid phase carriers (a nucleic acid precipitation agent), one or more steps can be removed from the standard purification process of nucleic acid from intact or whole cells. Furthermore, by providing solid phase carriers and a reagent that causes precipitation of the nucleic acid of the cell onto the solid phase carriers, one or more steps can be removed from the standard purification process of nucleic acid from cell lysates. The order in which the solid phase carriers and the reagent are combined with a cell or cell lysate is not critical. The solid phase carriers and the reagent that causes precipitation of the nucleic acid of the cell onto the solid phase carriers and/or lysis of the cells, can be combined with a cell or cell lysate sequentially (e.g., as separate components) or simultaneously (e.g., as a single component). When the solid phase carriers and the reagent are combined into a single component, the methods described herein allow for the addition of a single reagent to a cell or cell culture, followed by an incubation, a separation of a solid phase carrier and a selective elution to achieve separation of a cell's genomic nucleic acid from the cell's nucleic acid which has a molecular weight that is lower than the molecular weight of the genomic nucleic acid. No pH adjustments are required by the methods of the invention. The reduced number of steps provided by the reagents and methods described herein simplifies the automation of the nucleic acid purification process of cells or cell lysates.

Accordingly, the present invention relates to a method of separating genomic nucleic acid of a cell from nucleic acid having a molecular weight that is lower than the molecular weight of the genomic nucleic acid in the cell (e.g., plasmid DNA, episomal DNA, mitochondrial DNA, organelle DNA, viral DNA) comprising combining i) solid phase carriers (e,g., magnetic microparticles) whose surfaces have bound thereto a functional group (e.g., carboxyl group, amine group) which reversibly binds nucleic acid, ii) a cell and iii) a reagent, wherein the reagent causes lysis of the cell and precipitation of the nucleic acid of the cell onto the solid phase carriers, thereby producing a combination. The combination is maintained under conditions in which lysis of the cell occurs and the nucleic acid of the cell binds reversibly to the solid phase carriers; thereby producing solid phase carriers having nucleic acid of the cell bound thereto. The solid phase carriers are separated from the combination and contacted with an elution buffer (e.g., water).that causes elution (selective elution) of the nucleic acid having a lower molecular weight than the genomic nucleic acid from the solid phase carriers. The genomic nucleic acid remains bound to the solid phase carrier, thereby resulting in the separation of genomic nucleic acid of the cell from nucleic acid having a molecular weight that is lower than the molecular weight of the genomic nucleic acid in the cell.

In the method according to the present invention, the nucleic acid having a lower molecular weight is plasmid nucleic acid and the solid phase carriers comprise paramagnetic microparticles. The reagent may be an alcohol such as ethanol, isopropanol, polyalkylene glycol.

The present invention also relates to a method of separating genomic nucleic acid of a cell from nucleic acid having a molecular weight that is lower than the molecular weight of the genomic nucleic acid in the cell comprising combining i) solid phase carriers whose surfaces have bound thereto a functional group which reversibly binds nucleic acid, ii) a cell lysate and iii) a reagent, wherein the reagent causes precipitation of the nucleic acid of the cell lysate onto the solid phase carriers, thereby producing a combination. The combination is maintained under conditions in which the nucleic acid of the cell lysate binds reversibly to the solid phase carriers, thereby producing solid phase carriers having nucleic acid of the cell bound thereto. The solid phase carriers are separated from the combination and contacted with an elution buffer that causes elution of the nucleic acid having a lower molecular weight than the genomic nucleic acid from the solid phase carriers. The genomic nucleic acid remains bound to the solid phase carrier, thereby separating genomic nucleic acid of the cell from nucleic acid having a molecular weight that is lower than the molecular weight of the genomic nucleic acid in the cell.

### BRIEF DESCRIPTION OF THE DRAWINGS

Figure 1 is an illustration of the protocol for separation of genomic nucleic acid of a cell from nucleic acid having a lower molecular weight than the genomic nucleic acid in the cell.
Figure 2 is a 96-well Agarose gel which shows separation of *E. coli* genomic nucleic acid of a cell from a plasmid in the cell.
Figure 3 is a histogram of the Phred 20 (red) and Phred 30 (black) bases generated by the reads; the Y axis is number of reads, the X axis if Phred 20 binds in 50bp increments.
Figure 4 shows gDNA duplicates prepared from 50 ul horse blood.
Figure 5 shows the PicoGreen Analysis of 8 samples prepared gDNA from horse blood.
Figure 6 shows a gradient PCR of prepared gDNA from horse blood (using Y3B19 markers with an expected amplicon size of 225bp).

### DETAILED DESCRIPTION OF THE INVENTION

As described herein, the present invention provides methods in which a cell's genomic nucleic acid can be separated from the cell's nucleic acid which has a molecular weight that is lower than the molecular weight of the genomic nucleic acid (e.g., plasmid DNA) using a minimal number of steps. The separation can be performed on a cell culture directly without the need to pellet the cells. In addition, the methods described herein can be performed directly on a cell lysate without the need to clear the lysate of genomic nucleic acid using traditional methods (e.g., centrifugation, chemical treatment).

The present invention provides methods and reagents for isolating nucleic acids. The reagents described herein can be used to separate genomic nucleic acid of a cell (one or more) from nucleic acid having a molecular weight that is lower than the molecular weight of the genomic nucleic acid of the cell, by combining the cell with solid phase carriers and a reagent which causes lysis of the cell and precipitation of nucleic acid of the cell onto the solid phase carriers. Alternatively, the reagents described herein can be used to separate genomic nucleic acid of a cell lysate from nucleic acid having a molecular weight that is lower than the molecular weight of the genomic nucleic acid of the cell lysate, by combining the cell lysate with solid phase carriers and a reagent which causes precipitation of nucleic acid of the cell lysate onto the solid phase carriers. The nucleic acid having a molecular weight that is lower than the molecular weight of the genomic nucleic acid of the cell is then selectively eluted from the solid phase carriers.

As described herein, the method comprises binding the nucleic acid of a cell or cell lysate nonspecifically and reversibly to solid phase carriers (e.g., magnetic microparticles) having a functional group coated surface (e.g., carboxyl coated surface). The microparticles are then separated from the supernatant, for example, by applying a magnetic field to draw down the magnetic microparticles. The remaining solution, (e.g., supernatant) can then be removed, leaving the microparticles with the bound nucleic acid. Once separated from the supernatant, the microparticles can be contacted with an elution buffer that selectively elutes the nucleic acid having a molecular weight that is lower than the molecular weight of genomic nucleic acid of the cell. As a result, an elution buffer containing unbound nucleic acid (the cell's nucleic acid which has a lower molecular weight than the molecular weight of the cell's genomic nucleic acid) and magnetic microparticles to which genomic nucleic acid of the cell is still bound are produced. An elution buffer is a solution in which the concentration of a nucleic acid precipitating reagent is below the range required for binding of nucleic acid having a molecular weight that is lower than the molecular weight of genomic nucleic acid onto magnetic microparticles. In one embodiment, the eluent is water. In addition, sucrose (20%) and formamide (100%) solutions can be used to elute the nucleic acid. Elution of the nucleic acid from the microparticles occurs in thirty seconds or less when an elution buffer of low ionic strength, for example, water, is used. Once the bound nucleic acid has been eluted, the magnetic microparticles are separated from the elution buffer that contains the eluted nucleic acid. Preferably, the magnetic microparticles are separated from the elution buffer by magnetic means. Other methods known to those skilled in the art can be used to separate the magnetic microparticles from the supernatant. For example, filtration or centrifugation can be used.

The isolation of high quality nucleic acid preparations from starting solutions of diverse composition and complexity is a fundamental technique in molecular biology. As a result of the reagents and methods described herein, rapid and readily automatable methods of separating genomic nucleic acid from nucleic acid having a lower molecular weight than genomic nucleic acid are now available. Nucleic acids isolated by the disclosed methods can be used for molecular biology applications requiring high quality nucleic acids, such as the preparation of DNA sequencing templates, microinjection, transfection or transformation of mammalian cells, *in vitro* synthesis of RNAi hairpins, reverse transcription cloning, cDNA library construction, PCR amplification, and gene therapy research, as well as for other applications with less stringent quality requirements including, but not limited to, transformation, restriction endonuclease or microarray analysis, selective RNA precipitations, *in vitro* transposition, separation of multiplex PCR amplification products, preparation of DNA probes and primers and detemplating protocols.

The reagents and methods described herein can be used together with a variety of nucleic acid purification techniques, including those described in U.S. Pat. Nos. 5,705,628 and 5,898,071 as well as WO 99/58664.

In one embodiment, the present invention relates to a method of separating genomic nucleic acid of a cell from nucleic acid having a molecular weight that is lower than the molecular weight of the genomic nucleic acid (*e.g.*, plasmid DNA) in the cell, comprising combining i) solid phase carriers whose surfaces have bound thereto a functional group which reversibly binds nucleic acid, ii) a cell and iii) a reagent, wherein the reagent causes lysis of the cell and precipitation of the nucleic acid of the cell onto the solid phase carriers, thereby producing a combination. The combination is maintained under conditions in which lysis of the cell occurs and the nucleic acid of the cell binds reversibly to the solid phase carriers, thereby producing solid phase carriers having nucleic acid of the cell bound thereto. The solid phase carriers are separated from the combination and contacted with an elution buffer that causes elution of the nucleic acid having a molecular weight that is lower than the molecular weight of the genomic nucleic acid from the solid phase carriers, but does not cause elution of the genomic nucleic acid from the solid phase carriers, thereby separating genomic nucleic acid of the cell from nucleic acid having a molecular weight that is lower than the molecular weight of the genomic nucleic acid in the cell.

In another embodiment, the present invention relates to a method of separating genomic nucleic acid of a cell from nucleic acid having a molecular weight that is lower than the molecular weight of the genomic nucleic acid in the cell, comprising combining i) solid phase carriers whose surfaces have bound thereto a functional group which reversibly binds nucleic acid, ii) a cell lysate and iii) a reagent, wherein the reagent causes precipitation of the nucleic acid of the cell lysate onto the solid phase carriers, thereby producing a combination. The combination is maintained under conditions in which the nucleic acid of the cell lysate binds reversible to the solid phase carriers, thereby producing solid phase carriers having nucleic acid of the cell bound thereto. The solid phase carriers are removed from the combination and contacted with an elution buffer that causes elution of the nucleic acid having a molecular weight that is lower than the genomic nucleic acid, but does not cause elution of the genomic nucleic acid from the solid phase carriers, thereby separating genomic nucleic acid of the cell from nucleic acid having a molecular weight that is lower than the molecular weight of the genomic nucleic acid in the cell.

The present invention further relates to a method of separating genomic nucleic acid of a cell from nucleic acid having a molecular weight that is lower than the molecular weight of the genomic nucleic acid in the cell, wherein the nucleic acid having a lower molecular weight is suitable for use in either manual or a high-throughput automated sequencing methods.

In one embodiment, the invention is a readily automatable method of isolating a plasmid DNA template for nucleotide sequencing. The use of the reagents described herein affords an alternative, and readily automatable, means of nucleic acid separation.

As used herein the term "separating" is intended to mean that the material in question exists in a physical milieu distinct from that in which it occurs in nature and/or has been completely or partially separated, isolated or purified from other nucleic acid molecules.

As used herein the terms "nucleic acid" and "nucleic acid molecule" are used synonymously with the term polynucleotides and they are meant to encompass DNA (*e.g.*, single-stranded, double-stranded, covalently closed, and relaxed circular forms), RNA (*e.g.*, single-stranded and double-stranded), RNA/DNA hybrids and polyamide nucleic acids (PNAs).

"Genomic nucleic acid" refers to the genomic or chromosomal nucleic acid present in a cell. Typically, the molecular weight of genomic or chromosomal nucleic acid is from about 500 kilobases (kb) (*e.g.*, mycoplasma) to about 500 gigabases (Gb). In particular embodiments, the molecular weight of genomic or chromosomal nucleic acid ranges from about 1000 kb to about 250 Gb (*e.g.*, onion); from about 10,000 kb to about 5 Gb; from about 100,000 kb to about 1 Gb; and from about 500,000 kb to 1,000,000 kb.

"Nucleic acid having a molecular weight that is lower than the molecular weight of the genomic nucleic acid in the cell" refers to nucleic acid other than genomic or chromosomal nucleic acid that is present in a cell and can be endogenous or exogenous nucleic acid. Nucleic acid having a molecular weight that is lower than the molecular weight of the genomic nucleic acid in the cell typically has a molecular weight between about 1 kb to about 250,000 kb (*e.g.*, BAC). In particular embodiments, nucleic acid having a molecular weight that is lower than the molecular weight of the genomic nucleic acid in the cell ranges from about 5 kb to about 100,000 kb; from about 100 kb to about 10,000 kb; and from about 1000 kb to about 5000 kb. As used herein "endogenous nucleic acid" (host cell nucleic acid) refer to nucleic acid that are present in the cell as the cell is obtained. "Exogenous nucleic acid" (foreign nucleic acid; recombinant nucleic acid) refer to nucleic acid that is not present in the cell as obtained (*e.g.*, transfected cell, transduced cell). Exogenous nucleic acid may be present in a cell as a result of being introduced into the cell or being introduced into an ancestor of the cell. The exogenous nucleic acid may be introduced directly or indirectly into the cell or an ancestor thereof by means known to one of ordinary skill in the art (*e.g.*, transformation or transfection). Examples of exogenous nucleic acid introduced into a cell include bacterial artificial chromosome (BAC), yeast artificial chromosomes (YAC), plasmids, cosmids, P1 vector and nucleic acid introduced due to an amplification process (*e.g.*, polymerase chain reaction (PCR)). As used herein the term "plasmid" refers to double stranded circular DNA species which originate from an exogenous source (*e.g.*, are introduced into a host cell) and which are capable of self-replication independent of host chromosomal DNA. Thus, the term encompasses cloned DNA produced from the replication of any of the above-mentioned vectors. Examples of vectors used to introduce nucleic acid into a cell include pUC, pOT, pBluescript, pGEM, pTZ, pBR322, pSC101, pACYC, SuperCos and pWE15. Alternatively, the exogenous nucleic acid may be introduced into the cell from a phage into which the nucleic acid has been packaged (*e.g.*, cosmid, P1). Additional examples of "nucleic acid having a molecular weight that is lower than the molecular weight of the genomic nucleic acid in the cell" include, but are not limited to, episomal nucleic acid, mitochondrial nucleic acid, organelle nucleic acid, RNA, siRNA, plastids, microchromosomes, organelle nucleic acid, primers, viral nucleic acid, bacterial nucleic acid and nucleic acid from other pathogens.

A "solid phase carrier" is an entity that is essentially insoluble under conditions upon which a nucleic acid can be precipitated. Suitable solid phase carriers for use in the methods of the present invention have sufficient surface area to permit efficient binding and are further characterized by having surfaces which are capable of reversibly binding nucleic acids. Suitable solid phase carriers include, but are not limited to, microparticles, fibers, beads and supports which have an affinity for nucleic acid and which can embody a variety of shapes, that are either regular or irregular in form, provided that the shape maximizes the surface area of the solid phase, and embodies a carrier which is amenable to microscale manipulations. In one embodiment, the solid phase carrier is paramagnetic, e.g., a paramagnetic microparticle (magnetically responsive). In another embodiment, the solid phase carrier includes a functional group coated surface. For example, the solid phase carrier can be an amine-coated paramagnetic microparticle, a carboxyl-coated paramagnetic microparticle, or an encapsulated carboxyl group-coated paramagnetic microparticle.

As used herein, "paramagnetic microparticles" refers to microparticles which respond to an external magnetic field (*e.g.*, a plastic tube or a microtiter plate holder with an embedded rare earth (*e.g.*, neodymium) magnet) but which demagnetize when the field is removed. Thus, the paramagnetic microparticles are efficiently separated from a solution using a magnet, but can be easily resuspended without magnetically induced aggregation occurring. Preferred paramagnetic microparticles comprise a magnetite rich core encapsulated by a pure polymer shell. Suitable paramagnetic microparticles comprise about 20-35% magnetite/encapsulation ratio. For example, magnetic particles comprising a magnetite/encapsidation ratio of about 23%, 25%, 28% 30% 32% or 34% are suitable for use in the present invention. Magnetic particles comprising less than about a 20% ratio are only weakly attracted to the magnets used to accomplish magnetic separations. Depending on the nature of the host cell, the viscosity of the cell growth and the nature of the vector (e.g. high or low copy) paramagnetic microparticles comprising a higher percentage of magnite should be considered. The use of encapsulated paramagnetic microparticles, having no exposed iron, or Fe₃O₄ on their surfaces, eliminates the possibility of iron interfering with polymerase function in certain downstream manipulations of the isolated DNA. However the larger the magnetite core the higher the chance of encapsulation leakage (e*.*g*.*, release of iron oxides). Suitable paramagnetic microparticles for use in the instant invention can be obtained for example from Bangs Laboratories Inc., Fishers, IN (e.g., estapor® carboxylate-modified encapsulated magnetic microspheres), Agencourt Biosciences and Seradyn.

Suitable paramagnetic microparticles should be of a size that their separation from solution, for example by magnetic means or by filtration, is not difficult. In addition, preferred paramagnetic microparticles should not be so large that their surface area is minimized or that they are not suitable for microscale manipulation. Suitable sizes range from about 0.1µ mean diameter to about 100µ mean diameter. A preferred size is about 1.0µ mean diameter. Suitable magnetic microparticles are commercially available from PerSeptive Diagnostics and are referred to as BioMag COOH.

As used herein, the term "functional group-coated surface" refers to a surface which is coated with moieties which reversibly bind nucleic acid (e.g., DNA, RNA or polyamide nucleic acids (PNA)). One example is a surface which is coated with moieties which each have a free functional group which is bound to the amino group of the amino silane or the microparticle; as a result, the surfaces of the microparticles are coated with the functional group containing moieties. The functional group acts as a bioaffinity adsorbent for precipitated nucleic acid (*e.g.*, polyalkylene glycol precipitated DNA). In one embodiment, the functional group is a carboxylic acid. A suitable moiety with a free carboxylic acid functional group is a succinic acid moiety in which one of the carboxylic acid groups is bonded to the amine of amino silanes through an amide bond and the second carboxylic acid is unbonded, resulting in a free carboxylic acid group attached or tethered to the surface of the paramagnetic microparticle. Carboxylic acid-coated magnetic particles are commercially available from PerSeptive Diagnostics (BioMag COOH). Suitable solid phase carriers having a functional group coated surface that reversibly binds nucleic acid molecules are for example, magnetically responsive solid phase carriers having a functional group-coated surface, such as, but not limited to, amino-coated, carboxyl-coated and encapsulated carboxyl group-coated paramagnetic microparticles.

Appropriate starting material include cells (intact or whole cells), cell lysates (cells in growth or culture media) and lysates prepared from such cells. Appropriate starting material include cells obtained from either mammalian (i.e., human, primate, equine, canine, feline, bovine, murine) tissue or body fluids and lysates prepared from such cells. Thus, any type of cell having genomic nucleic acid and nucleic acid having a molecular weight lower than the molecular weight of the genomic nucleic acid can be used. Examples of cells for use in the methods of the present invention include, but are not limited to, mammalian cells (*e.g.*, blood cells, such as whole blood cells), bacterial cells (*e.g.*, *E*. *Coli* such as DH5α, DH10B, DH12S, C600 or XL-1 Blue), yeast cells, plant cells, tissue cells (cells from, for example, *C*. *elegans,* mouse tails, human biopsies) and host cells containing exogenous nucleic acid (e.g., recombinant DNA, bacterial DNA or replicative form DNA) which is targeted for isolation from host cell chromosomal DNA and other host cell biomolecules. Alternatively, the starting material can be lysates prepared from such cells.

As used herein a "host cell" is any cell into which exogenous nucleic acid can be introduced, thereby producing a host cell which contains exogenous nucleic acid, in addition to host cell nucleic acid. As used herein the terms "host cell nucleic acid" and "endogenous nucleic acid" refer to nucleic acid species (e.g., genomic or chromosomal nucleic acid) that are present in a host cell as the cell is obtained. As used herein, the term "exogenous" refers to nucleic acid other than host cell nucleic acid (*e.g.*, plasmid); exogenous nucleic acid can be present into a host cell as a result of being introduced in the host cell or being introduced into an ancestor of the host cell. Thus, for example, a nucleic acid species which is exogenous to a particular host cell is a nucleic acid species which is non-endogenous (not present in the host cell as it was obtained or an ancestor of the host cell). Appropriate host cells include, but are not limited to, bacterial cells, yeast cells, plant cells and mammalian cells.

As described herein, an advantage of the present invention is that a cell's genomic nucleic acid can be separated from the cell's nucleic acid which has a molecular weight that is lower than the molecular weight of the genomic nucleic acid using a minimal number of steps. As described herein, the separation can be performed on a cell culture directly without the need to pellet the cells. In addition, the method can be performed on a cell lysate, wherein the methods of the present invention make it unnecessary to clear the lysate in order to separate genomic nucleic acid of a cell from nucleic acid having a molecular weight that is lower than the molecular weight of the genomic nucleic acid.

As used herein, a "lysate" is a solution containing cells which contain genomic nucleic acid and nucleic acid having a lower molecular weight than genomic nucleic acid and whose cell membranes have been disrupted by any means with the result that the contents of the cell, including the nucleic acid therein, are in solution. A "cleared lysate" is a lysate in which the chromosomal or genomic nucleic acid, proteins and membranes of the cell have been removed such as by chemical treatment or centrifugation of the lysate. Cells are lysed using known methods, thereby preparing a mixture suitable for use with the method of the instant invention. For example, cells can be lysed using chemical means (*e.g.*, alkali or alkali and anionic detergent treatment), isotonic shock, or physical disruption (*e.g.*, homogenization).

The term "lysed host cell suspension", as used herein, refers to a suspension comprising host cells whose membranes have been disrupted by any means (e.g., chemical, such as alkali or alkali and anionic detergent treatment, isotonic shock, or physical disruption by homogenization); such a suspension is a mixture of host cell biomolecules, cellular components and disrupted membrane debris. In one embodiment, a lysed host cell suspension suitable for use in the instant invention is prepared by contacting host cells with an alkali and anionic detergent (e.g., sodium dodecyl sulphate (SDS)) solution (e.g., 0.2 N NaOH, 1% SDS). Optionally, lysozyme could be included in the lysis buffer. The presence of an anionic detergent in the lysing solution functions to produce an anti-protein environment by neutralizing the effective charge of the proteins, thereby minimizing their attraction to the surfaces of the functional group-coated paramagnetic microparticles. In one embodiment, the lysed host cell suspension is non-neutralized. Optionally, RNAse can be added to the host cell lysate to degrade host cell RNA, thereby allowing DNA to bind to the magnetic microparticles free, or essentially free, from RNA.

According to the methods of the present invention, a cell is combined with solid phase carriers and a reagent, wherein the reagent causes the nucleic acids of the cell to bind non-specifically and reversibly to the solid phase carriers.

"Non-specific nucleic acid binding" refers to binding of different nucleic acid molecules with approximately the same affinity to magnetic microparticles, despite differences in the nucleic acid sequence or size of the different nucleic acid molecules. As used herein, "facilitated adsorption" refers to a process whereby a precipitating reagent, (e.g., a poly-alkyelene glycol) is used to promote the precipitation and subsequent adsorption of a species of DNA molecules, which were initially in mixture, onto the surface of a solid phase carrier. The resulting reversible interaction is distinct from, for example, an interaction between two binding partners (e.g., streptavidin/biotin, antibody/antigen or a sequence-specific interaction), which are conventionally utilized for the purpose of isolating particular biomolecules based on their composition or sequence.

A "nucleic acid precipitating reagent" or "nucleic acid precipitating agent" is a composition that causes the nucleic acid of a cell to go out of solution. Suitable precipitating agents include alcohols (*e.g.*, short chain alcohols, such as ethanol or isopropanol) and a poly-OH compound (*e.g.*, a polyalkylene glycol). The nucleic acid precipitating reagent can comprise one or more of these agents. The nucleic acid precipitating reagent is present in sufficient concentration to nonspecifically and reversibly bind the nucleic acid of the cell onto the solid phase carriers.

Appropriate alcohol (*e.g.*, ethanol, isopropanol) concentrations (final concentrations) for use in the methods of the present invention are from about 40% to about 60%; from about 45% to about 55%; and from about 50% to about 54%.

Appropriate polyalkylene glycols include polyethylene glycol (PEG) and polypropylene glycol. Suitable PEG can be obtained from Sigma (Sigma Chemical Co., St. Louis MO., Molecular weight 8000, Dnase and Rnase fee, Catalog number 25322-68-3) The molecular weight of the polyethylene glycol (PEG) can range from about 6000 to about 10,000, from about 6000 to about 8000, from about 7000 to about 9000, from about 8000 to about 10,000. In a particular embodiment PEG with a molecular weight of about 8000 is used. In general, the presence of PEG provides a hydrophobic solution which forces hydrophilic nucleic acid molecules out of solution. In one embodiment, the PEG concentration is from about 5% to about 20%. In other embodiments, the PEG concentration ranges from about 7% to about 18%; from about 9% to about 16%; and from about 10% to about 15%.

As described above, in the embodiment in which the starting material is a cell the reagent is formulated to cause the lysis of a cell. A variety of lysis components can be used to cause the disruption of a membrane (such as alkali, alkali and anionic detergent treatment, or isotonic shock). In one embodiment, the lysis component of the reagent is an alkali (NaOH) and/or an anionic detergent (e.g., sodium dodecyl sulphate (SDS)) solution (e.g., final concentration of 0.2 N NaOH, 1% SDS when added to a cell). Optionally, lysozyme could be included in the lysis component of the first reagent. The presence of an anionic detergent in the lysis component functions to produce an anti-protein environment by neutralizing the effective charge of the proteins, thereby minimizing their attraction to the surfaces of the solid phase carrier (*e.g.*, a functional group-coated paramagnetic microparticle). Optionally, RNAse (*e.g.*, 1.75ng/ul RNAse/ddH₂O) can be added to the lysis component to degrade host cell RNA, thereby allowing DNA to bind to the solid phase carrier free, or essentially free, from RNA. The necessity of including a RNAse step will largely be determined by the size of the nucleic acid species that is targeted for isolation in the particular nucleic acid precipitation that is being performed. For example, if the conditions selected for isolation are appropriate for isolating nucleic acids comprising at least 4,000 base pairs, then it is unlikely that RNA species will be an appreciable contaminant.

The reagent used in the methods described above is useful for isolating a nucleic acid from a cell. This reagent contains a nucleic acid precipitating agent and a solid phase carrier, and can also be formulated to cause lysis of a cell(s). The nucleic acid precipitating agent is of sufficient concentration to precipitate the nucleic acid of the cell. The solid phase carrier in this reagent contains a surface that binds nucleic acid of the cell. The components of the reagent can be contained in a single reagent or as separate components. The components can be combined simultaneously or sequentially with cells. The order in which the elements of the combination are combined is not critical. The nature and quantity of the components contained in the reagent are as described in the methods above. The reagent may be formulated in a concentrated form, such that dilution is required to obtain the functions and or concentrations described in the methods herein.

Optionally, salt may be added to the reagent to cause precipitation of the nucleic acid of the cell onto the solid phase carriers. Suitable salts which are useful for facilitating the adsorption of nucleic acid molecules targeted for isolation to the magnetically responsive microparticles include sodium chloride (NaCl), lithium chloride (LiCl), barium chloride (BaCl₂, potassium (KCl), calcium chloride (CaCl₂, magnesium chloride (MgCl₂) and cesium chloride (CsCl). In one embodiment, sodium chloride is used. In general, the presence of salt functions to minimize the negative charge repulsion of the nucleic acid molecules. The wide range of salts suitable for use in the method indicates that many other salts can also be used and suitable levels can be empirically determined by one of ordinary skill in the art. The salt concentration can be from about 0.1M to about 0.5M; from about 0.15M to about 0.4M; and from about 0.2M to about 0.4M.

At high salt concentrations (*e.g.*, synonymous with high ionic strengths) suitable paramagnetic microparticles will adsorb DNA fragments of all sizes. The term "high salt concentration" refers to salt concentrations greater than about 0.5 M. At "low salt concentrations" (or low ionic strenghs), which as used herein connotes concentrations less than about 0.2 M, essentially no DNA, of any size, will be precipitated even in the presence of a PEG concentration that is as high as 12% (weight/volume) (Lis, John T, Methods in Enzymology 65: 437-353 (1980). Additional components may be added to the reagent. In one embodiment, RNAse is added to the nucleic acid precipitating agent.

According to the methods of the invention, the isolation of the nucleic acid molecules of the cell is accomplished by removing the nucleic acid-coated solid phase carrier from the combination. The solid phase carrier (*e.g.*, a paramagnetic microparticle) can be recovered from the first combination, for example, by vacuum filtration, centrifugation, or by applying a magnetic field to draw down the solid phase carrier (*e.g*., a paramagnetic microparticle). Paramagnetic microparticles are preferably separated from solutions using magnetic means, such as applying a magnet field of at least 1000 Gauss. However, other methods known to those skilled in the art can be used to remove the magnetic microparticles from the supernatant (e.g., vacuum filtration or centrifugation). The remaining solution can then be removed, leaving solid phase carriers having the nucleic acid of the cell adsorbed to their surface. Once separated from the mixture, the nucleic acid having a lower molecular weight than the molecular weight of the genomic nucleic acid of the cell which is adsorbed to the solid phase carrier can be recovered by contacting the solid phase carrier with a suitable elution buffer. As a result, 1) a solution comprising the nucleic acid molecules having a molecular weight that is lower than the molecular weight of the genomic nucleic acid in the elution buffer and 2) solid phase carriers to which is bound genomic nucleic acid of the cell, are produced.

A suitable "elution buffer" for use in the methods of the present invention is a buffer that selectively elutes a cell's nucleic acid which has a molecular weight that is lower than the molecular weight of the cell's genomic nucleic acid. In one embodiment, a suitable elution buffer for use in the present invention can be water or any aqueous solution in which the nucleic acid precipitating agent (*e.g.*, isopropanol and/or PEG) concentration is below the concentration required for binding of as cell's nucleic acid which has a molecular weight that is lower than the molecular weight of the cell's genomic nucleic acid to the solid phase carrier, as discussed above. For example, useful buffers include, but are not limited to, TRIS-HCl,Tris acetate, sucrose (20%) and formamide (100%) solutions. Elution of a cell's nucleic acid which has a molecular weight that is lower than the molecular weight of the cell's genomic nucleic acid from the solid phase carrier can occur quickly (e.g., in thirty seconds or less) when a suitable low ionic strength elution buffer is used. Once the bound DNA has been eluted, the solid phase carrier, to which is bound the cell's genomic nucleic acid, is separated from the elution buffer.

Optionally, impurities (e.g., host cell components, proteins, metabolites, chemicals or cellular debris) can be removed by washing the paramagnetic microparticles with target nucleic acid bound thereto (e.g., by contacting the microparticles with a suitable wash buffer solution) before separating the microparticle-bound nucleic acid from the solid phase carriers. As used herein, a "wash buffer" is a composition that dissolves or removes impurities either bound directly to the microparticle, or associated with the adsorbed nucleic acid, but does not solubilize the nucleic acid absorbed onto the solid phase. The pH and solute composition and concentration of the wash buffer can be varied according to the types of impurities which are expected to be present. For example, ethanol (*e.g.*, 70%) exemplifies a preferred wash buffer useful to remove excess PEG and salt. The magnetic microparticles with bound nucleic acid can also be washed with more than one wash buffer solution. The microparticles can be washed as often as required (e.g., three to five times) to remove the desired impurities. However, the number of washings is preferably limited in order to minimize loss of yield of the bound nucleic acid. A suitable wash buffer solution has several characteristics. First, the wash buffer solution must have a sufficiently high salt concentration (a sufficiently high ionic strength) that the nucleic acid bound to the magnetic microparticles does not elute off of the microparticles, but remains bound to the microparticles. A suitable salt concentrations is greater than about 0.1 M and is preferably about 0.5M. Second, the buffer solution is chosen so that impurities that are bound to the nucleic acid or microparticles are dissolved. The pH and solute composition and concentration of the buffer solution can be varied according to the types of impurities which are expected to be present. Suitable wash solutions include the following: 0.5 x 5 SSC; 100 mM ammonium sulfate, 400 mM Tris pH 9,25 mM MgCl₂ and 1% bovine serum albumin (BSA); and 0.5M NaCl. In one embodiment, the wash buffer solution comprises 25 mM Tris acetate (pH 7.8),100 mM potassium acetate (KOAc), 10 mM magnesium acetate (Mg₂OAc), and 1 mM dithiothreital (DTT). In another embodiment, the wash solution comprises 2% SDS, 10% Tween and/or 10% Triton.

The isolation of high quality nucleic acid preparations from starting solutions of diverse composition and complexity is a fundamental technique in molecular biology. Thus, as a result of the work described herein, novel and readily automatable methods of separating nucleic acid molecules having a molecular weight that is lower than the molecular weight of genomic nucleic acid are now available. In one embodiment, the reagent is added to the cell by a multisample transfer device. In another embodiment, the first reagent is added simultaneously to a plurality of samples, e.g., at least 6, 12, 24, 96, 384, or 1536 samples, each sample containing one or more cells. In another embodiment, the first reagent is sequentially delivered to a plurality of samples (*e.g.*, at least 6, 12, 24, 96, 384, or 1536 samples) each sample containing one or more cells. The invention includes methods of analyzing a plurality of nucleic acid samples. The methods include providing a plurality of nucleic acid samples isolated by a method described herein and analyzing the samples, e.g., performing sequence analysis on the samples.

In another embodiment, the isolated nucleic acid of one or a plurality of samples is subjected to further analysis (*e.g.*, sequence analysis). Nucleic acids isolated by the disclosed method can be used for molecular biology applications requiring high quality nucleic acids, for example, the preparation of DNA sequencing templates, the microinjection, transfection or transformation of mammalian cells, the *in vitro* synthesis of RNA probes, reverse transcription cloning, cDNA library construction, PCR amplification, or gene therapy research, as well as for other applications with less stringent quality requirements including, but not limited to, transformation, restriction endonuclease or microarray analysis, selective RNA precipitations, in vitro transposition, separation of multiplex PCR amplification products, *in vitro* siRNA, RNAi hairpins, preparation of DNA probes and primers and detemplating protocols.

### EXEMPLIFICATION

### Example 1 One embodiment of a protocol for separation of nucleic acid having a lower molecular weight than genomic nucleic acid in a cell

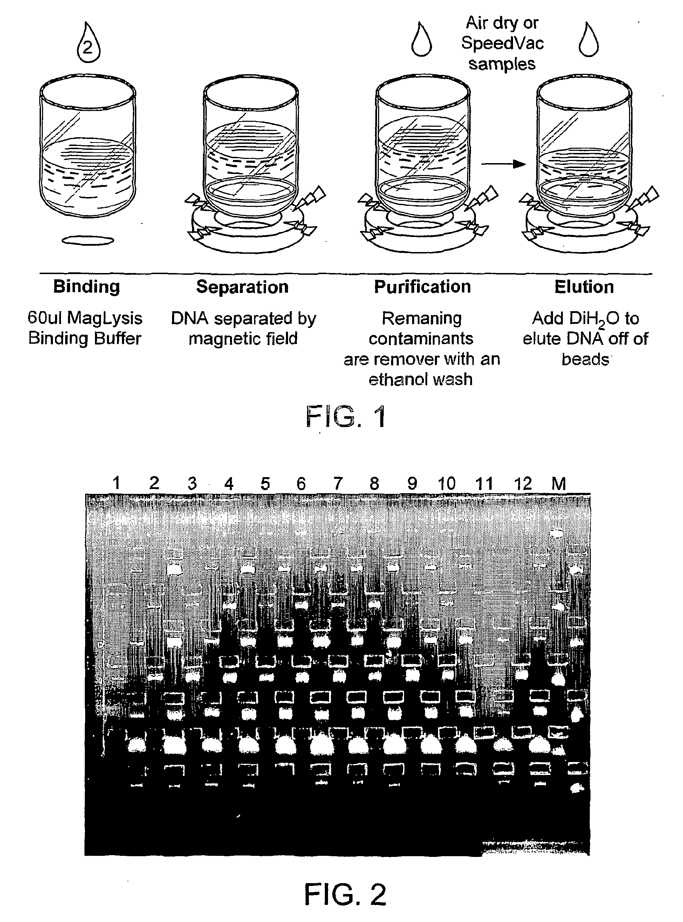

The following protocol is illustrated in Figure 1.

### Growth of Bacterial Cultures

1. Pipette 200 µL of 2xYT bacterial growth media containing the appropriate antibiotic into each well of a 300 µl Costar 96 well round bottom plate (Cat.# 3750).
2. Innoculate each well with a single plasmid containing *E. coli* bacterial colony (DH10B, DH5alpha: Invitrogen). Growth cultures can be innoculated directly from agar lawns or from glyceral stocks.
3. Cover the plate with a porous seal and shake vigorously (*e.g.*, 300 rpm) at 37°C for 16 hours.

### Purification Procedure

1. Add 60 µL of paramagnetic lysis buffer (0.4N NaOH, 2% SDS, 0.0016% solids Agencourt COOH magnetic microparticles) solution to each well of the source plate and shake or tip mix.
2. Add 60 µL of Wash A solution (100% isopropanol) to the samples. Perform 15 tip mixes once the Wash A solution is added. Tip mixing conditions may vary depending on the tip orifice
3. Place source plate onto a magnetic SPRIplate96-R(TM) (cat.# Agencourt Biosciences) for 5 minutes to separate beads from solution.
4. Aspirate and discard the cleared solution from the destination plate while it is situated on a SPRIplate96-R (TM) (RING Shaped magnets).
5. Dispense 200 µL of Wash B solution (70% ethanol) into each well of the Destination Plate as a wash solution.
6. Remove ethanol wash solution and repeat step four times.
7. Dry the destination plate at 37°C for 30 minutes.
8. Add 40 µL of elution buffer (ddH20 + 1.75 ng/µl RNAse A) to each well of the plate and shake. Reagent grade water is the recommended elution buffer, Vortex or shake the plate after adding elution buffer or wait 10 minutes for the elution to occur.

Sequencing Procedure:
For 1/24th x BigDye reaction add:
   60µl of purified DNA
   1 µl BigDye Cocktail
BigDye Cocktail
   5 parts BigDye Terminator
   1 part 200 µm M13-21 primer
   4 parts 15 x BigDye Buffer
Cycle Sequence
   95°C for 2 minutes
   50 cycles of (54°C for 15 seconds, 60°C for 2.5 minutes, 95°C for 5 seconds)
   4°C until cleanup

Purify sequencing reactions using either standard EtOH precipitation of Agencourt CleanSeq kit (cat. # 000121 and 000222).

Elute Precipitation of CleanSeq product in 30 µl of ddH2O and place on an ABI 3700 or 3730 for sequencing detection.

Over 11,000 reads sequences using POP5 polymer on the ABI 3700 produced 90% pass rates (passing read must average phred 20 from bp 100-300), and 625 Phred20 bases.

### Example 2 Isolation of exogenous nucleic acid having a lower molecular weight than genomic nucleic acid in bacterial cells

### Methods and Materials

### Cloning and purification:

Chimpanzee genomic DNA was sheared, end repaired with T4 polymerase and Klenow (NEB), and cloned in pOT bacterial vector. DH10B cells (Invitrogen) were electroporated and plated on 25 ug/ml chloramphenicol agar and grown overnight. Colonies were picked with a Gentix Qpix into 200ul of 2XYT, 50ug/ml Chloramphenicol broth and grown for 16 hours. The clones were purified in the growth plate on a Beckman FX robotic platform.

### Purification Process

Comparing method described herein (OneStep Prep) to the method described in U.S. Patent No. 6,534,262 (McPrep).
24 samples were purified using two different purification methods; McPrep and the single step purification method described herein. Controlled samples were loaded on an agarose gel to compare recovery (Figure 2).
60ul of paramagnetic lysis buffer (0.4N NaOH, 2% SDS, 160mg/liter Seradyn COOH paramagnetic beads) in addition to 60 ul of 100% isopropanol (or 100ul 40% PEG) was added to the cell culture and tip mixed 15 times. Samples were separated for 6 minutes on an Agencourt Magnet Plate (1000 gauss). Supernatant was removed and the separated beads were rinsed 5 times with 70% ethanol. After elution with 1.75ng/ul RNAse A/ddH2O (Sigma), samples were run on a 96 E-Gel(Invitrogen) to estimate relative DNA recovery (Figure 2). Pico Green Analysis (Molecular Probes) was also run on the samples and average DNA recovery was 20ng/ul. Average conductivity of the samples were recorded to estimate any salt contamination from the growth broth by pooling all 40ul of eluent from 10 wells. Conductivity was less than 20us/cm.

### Sequencing Reaction Setup:

Samples where eluted in 40ul of 1.75ng/ul RNAse/ddH20 and robotically agitated for 20cycles of 5cm at 2Hz. 3ul of DNA was aspirated and placed into 0.5ul (1/3X BigDye) reagent plus 3 pmole of -21 primer. Reactions were overlayed with mineral oil and cycle sequenced in a 384 well plate. Samples were cycle sequenced according to the manufacturers recommendations. Sequencing reactions were purified with Agencourt's CleanSeq kit and eluted in 15ul of ddH20 and heat sealed prior to loading on the ABI 3700.

### Detection:

DNA sequencing Data generated using ABI3700 sequencing platform and 1/48thX BigDye V3.1 Sequencing Chemistry and POP5 polymer. Electrophoresis was performed at 5800V with a 30 second injection.

### Results:

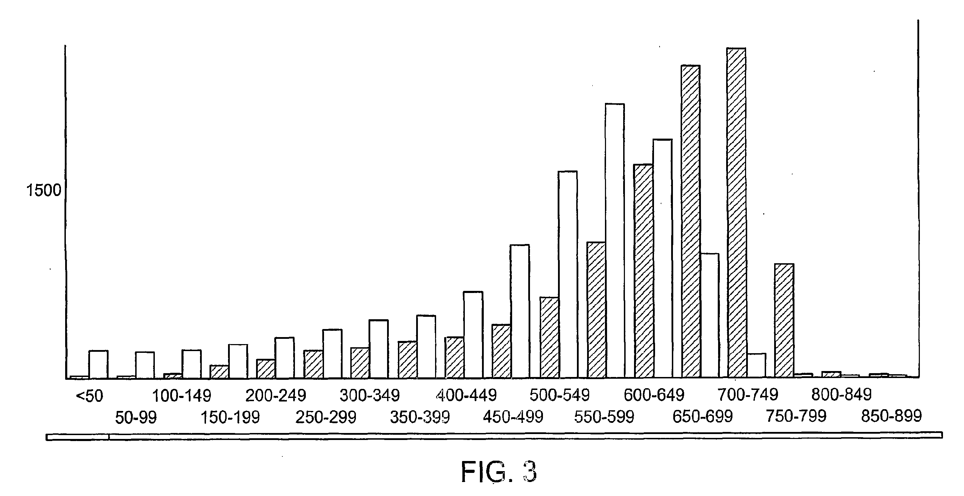

12672 sequencing reads were generated from 6336 purified samples (Table 1). The high Pass Rates, high Signal Intensity and high Average Phred 20 base pairs (Ewing et al., Genome Research, 8:175-185 (1998)) obtained are exceptional for 48 fold diluted BigDye sequencing chemistry. A histogram of the read performance is plotted in Figure 3.

Figure 2 shows a 96 well Agarose gel with 13 columns by 8 rows. The 13^{th} column is 200ng pGEM 3.2kb vector (Promega). Positive electrode is at the bottom of the picture. Prep samples are eluted in 40ul of various elution buffers and 10ul loaded on the gel. One can see a 3-5kb plasmid on the gels with no sign of *E. coli* genomic DNA remaining in the Agarose wells. RNA can be seen in wells that were not eluted in 1.75 ng/ul of RNAse (row F).

One can see more plasmid DNA is recovered using the single step method versus using the McPrep method (Row A vs Row B & Row C vs Row D). This is expected as the McPrep method requires *E. coli* genomic DNA to be bound to one set of beads and the plasmid enriched supernatant to be removed to another plate for binding to a second mixture. This supernatant removal step makes it difficult to aspirate 100% of the plasmid supernatant without disturbing the genomic DNA magnetic beads, so a loss of DNA is expected.

| | |
|---|---|
| Row A: | 10ul/40ul McPrep |
| Row B: | 10ul/40ul OneStep Prep |
| Row C: | 10ul/40ul McPrep |
| Row D: | 10ul/40ul OneStep Prep |
| Row E & F: | 10ul/40ul OneStep with and without RNAse |
| Row G: | 10ul/40ul McPrep without RNAse |

3ul eluted plasmid DNA was used for BigDye sequencing. Eletropherogram read quality was determined by running Phred (Ewing et al., Genome Research, 8:175-185 (1998)). Phred 30 bases have an error rate of 1 in 1000, Phred 40 bases have an error rate 1 in 10,000. Counting the number of phred 20 bases that each read produces is a standard sequencing quality metric (Table).
Pass Rate is defined as the number of reads meeting the PASS criteria/Total Reads
P30 = Average Number of Phred 30 per 384 well plate
P20 = Average Number of Phred 20 per 384 well plate
CP20= Average Number of Contiguous Phred 20s per 384 well plate
P 15= Average Number of Phred 15 per 384 well plate
Qual = Average Phred scores throughout the whole read: Averaged over 384 well plate
PASS criteria- A read must average Phred20 quality in a 200bp window from bp 100 to bp 300.
SigA = Average Relative Fluorescent Units in the A channel for the 96 reads.
SigG= Average Relative Fluorescent Units in the G channel for the 96 reads
SigC= Average Relative Fluorescent Units in the C channel for the 96 reads
SigT= Average Relative Fluorescent Units in the T channel for the 96 reads

| Seq Barcode | Pass | Total | Pass % | P30 | P20 | CP20 | P15 | Qual | Length | Sig A | Sig G | Sig C | Sig T |
|---|---|---|---|---|---|---|---|---|---|---|---|---|---|
| 000048032741 (KF) | 351 | 384 | 91.41 | 576 | 669 | 540 | 706 | 47 | 763 | 188 | 134 | 177 | 187 |
| 000028713241 (KN) | 360 | 384 | 93.75 | 528 | 626 | 482 | 670 | 46 | 743 | 149 | 118 | 161 | 158 |
| 000028713341 (KP) | 345 | 384 | 89.84 | 534 | 625 | 489 | 663 | 46 | 736 | 51 | 33 | 46 | 50 |
| 000028713641 (HK) | 345 | 384 | 89.84 | 538 | 620 | 509 | 660 | 44 | 744 | 93 | 64 | 85 | 103 |
| 000028714941 (GQ) | 353 | 384 | 91.93 | 552 | 639 | 515 | 677 | 43 | 741 | 202 | 151 | 191 | 208 |
| 000028715041 (KF) | 336 | 384 | 87.50 | 555 | 654 | 520 | 694 | 46 | 754 | 197 | 125 | 168 | 186 |
| 000048032841 (KF) | 350 | 384 | 91.15 | 568 | 659 | 527 | 696 | 46 | 754 | 165 | 114 | 146 | 162 |
| 000028712941 (KN) | 357 | 384 | 92.97 | 588 | 681 | 560 | 717 | 47 | 778 | 188 | 156 | 173 | 197 |
| 000028713041 (KH) | 369 | 384 | 96.09 | 585 | 670 | 545 | 705 | 47 | 768 | 94 | 56 | 63 | 82 |
| 000028713141 (KJ) | 341 | 384 | 88.80 | 538 | 627 | 521 | 663 | 47 | 726 | 113 | 79 | 110 | 148 |
| 000028713441 (KP) | 341 | 384 | 88.80 | 569 | 665 | 534 | 703 | 46 | 760 | 115 | 75 | 98 | 114 |
| 000028713541 (KN) | 339 | 384 | 88.28 | 528 | 632 | 487 | 677 | 46 | 755 | 268 | 217 | 295 | 292 |
| 000028713741 (GZ) | 347 | 384 | 90.36 | 549 | 639 | 515 | 677 | 44 | 741 | 165 | 117 | 143 | 146 |
| 000028713941 (HK) | 349 | 384 | 90.89 | 548 | 638 | 522 | 676 | 45 | 740 | 177 | 121 | 148 | 177 |
| 000028714141 (KN) | 352 | 384 | 91.67 | 498 | 598 | 447 | 643 | 45 | 727 | 108 | 83 | 113 | 114 |
| 000028714241 (KK) | 347 | 384 | 90.36 | 548 | 646 | 516 | 685 | 46 | 747 | 120 | 83 | 99 | 127 |
| 000028714441 (GY) | 350 | 384 | 91.15 | 529 | 621 | 503 | 660 | 46 | 727 | 138 | 98 | 124 | 135 |
| 000028714541 (GQ) | 343 | 384 | 89.32 | 525 | 620 | 483 | 663 | 43 | 738 | 194 | 118 | 182 | 184 |
| 000028714641 (KF) | 311 | 384 | 80.99 | 540 | 638 | 481 | 678 | 45 | 747 | 146 | 89 | 119 | 141 |
| 000028714741 (XB) | 351 | 384 | 91.41 | 488 | 612 | 371 | 661 | 38 | 726 | 170 | 120 | 128 | 178 |
| 000028714841 (KF) | 333 | 384 | 86.72 | 569 | 666 | 527 | 706 | 47 | 759 | 205 | 122 | 161 | 191 |
| 000028715141 (HH) | 363 | 384 | 94.53 | 536 | 618 | 507 | 652 | 46 | 712 | 191 | 129 | 157 | 201 |
| 000028715541 (GQ) | 358 | 384 | 93.23 | 532 | 619 | 505 | 660 | 43 | 731 | 110 | 86 | 102 | 110 |
| 000028715641 (KR) | 362 | 384 | 94.27 | 530 | 612 | 495 | 645 | 46 | 710 | 51 | 45 | 65 | 65 |
| 000028712841 (HG) | 361 | 384 | 94.01 | 537 | 634 | 504 | 675 | 44 | 741 | 360 | 220 | 335 | 339 |
| 000028714041 (KF) | 349 | 384 | 90.89 | 542 | 637 | 499 | 677 | 45 | 746 | 77 | 49 | 61 | 75 |
| 000028715241 (GW) | 359 | 384 | 93.49 | 497 | 580 | 462 | 618 | 43 | 699 | 132 | 67 | 102 | 117 |
| 000028715441 (HH) | 345 | 384 | 89.84 | 487 | 570 | 450 | 610 | 43 | 696 | 104 | 70 | 81 | 109 |
| 000028715741 (KS) | 354 | 384 | 92.19 | 464 | 546 | 393 | 585 | 45 | 675 | 39 | 29 | 39 | 38 |
| 000028713841 (HA) | 358 | 384 | 93.23 | 534 | 623 | 507 | 660 | 46 | 721 | 126 | 96 | 144 | 167 |
| 000028712741 (GR) | 326 | 384 | 84.90 | 434 | 520 | 401 | 561 | 41 | 674 | 76 | 53 | 64 | 73 |
| 000028714341 (GR) | 328 | 384 | 85.42 | 481 | 570 | 446 | 613 | 42 | 704 | 99 | 70 | 84 | 96 |
| 000028715341 (GR) | 334 | 384 | 86.98 | 478 | 563 | 455 | 603 | 42 | 698 | 114 | 69 | 84 | 99 |
| | 11467 | 12672 | 90.49 | 531 | 623 | 492 | 662 | 45 | 733 | 143 | 99 | 129 | 145 |

Figure 3 is a Histogram of the Phred 20 (red) ad Phred30 (black) bases generated by the reads. Y Axis is number of Reads, X axis is phred20 bins in 50bp increments.

### Example 3 Isolation of nucleic acid having a lower molecular weight than genomic nucleic acid in horse whole blood cells

### Materials and Methods

### Source

Horse whole blood was obtained in 1:1 ratio with Alsevers anti-coagulant (2.05% dextrose, 0.5% sodium citrate, 0.055% citric acid, 0.42% sodium chloride).

### Purification Process

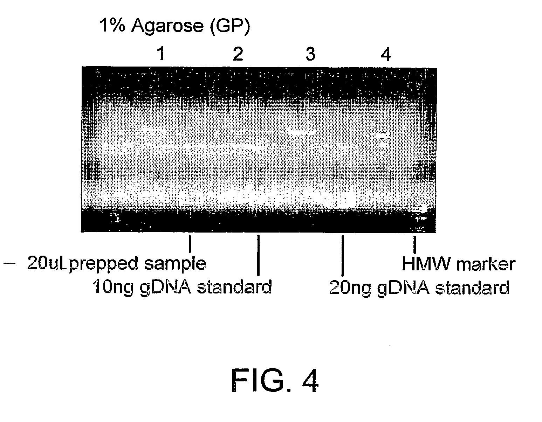

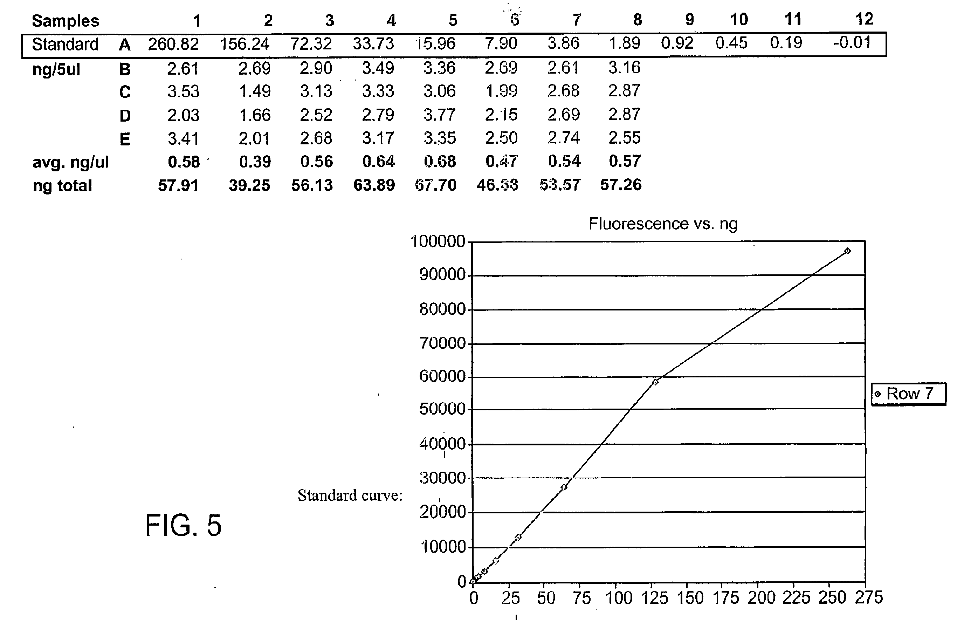

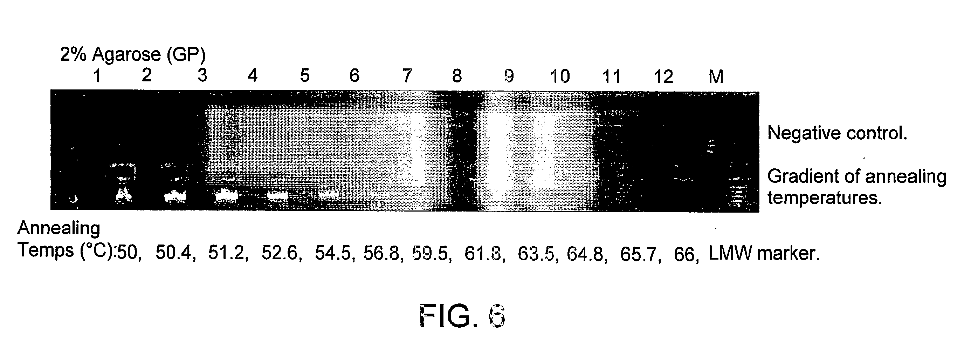

60ul paramagnetic lysis buffer (0.4NaOH, 2% SDS, 160mg/liter Seradyn COOH paramagnetic beads) in addition to 80ul of 100% isopropanol is added to the cell culture and tip mixed 15 times. Samples were separated for 15 minutes on an Agencourt Magnet Plate (1000 gauss). Supernatant was removed and the separated beads were rinsed 5 times with 70% ethanol. After elution with ddH₂O (Sigma), samples were run on a 96 E-Gel (Invitrogen) to estimate relative DNA recovery (Figure 4). Pico Green Analysis (Molecular Probes) was also run on the samples and average DNA recovery is 0.5ng/ul (Figure 5). DNA quality was verified in its applicability to the Polymerase Chain Reaction (PCR), a common downstream application (Figure 6).

Figure 4 shows gDNA duplicates prepped from 50 ul horse blood. Figure 5 shows the PicoGreen Analysis of 8 samples prepped gDNA. Figure 6 shows a gradient PCR of prepped gDNA above (using Y3B19 markers with an expected amplicon size of 225bp).

While this invention has been particularly shown and described with references to preferred embodiments thereof, it will be understood by those skilled in the art that various changes in form and details may be made therein without departing from the scope of the invention encompassed by the appended claims.

## Claims

1. A method of separating genomic nucleic acid of a cell from nucleic acid having a molecular weight that is lower than the molecular weight of the genomic nucleic acid in the cell comprising:
a) combining
i) solid phase carriers whose surfaces have bound thereto a functional group which reversibly binds nucleic acid,
ii) a cell and
iii) a reagent, wherein the reagent comprises a lysis component which causes lysis of the cell and a nucleic acid precipitation agent which causes precipitation of the nucleic acid of the cell onto the solid phase carriers;
thereby producing a combination;
b) maintaining the combination under conditions in which lysis of the cell occurs and the nucleic acid of the cell binds reversibly to the solid phase carriers, thereby producing solid phase carriers having nucleic acid of the cell bound thereto;
c) separating the solid phase carriers from the combination;
d) contacting the solid phase carriers with an elution buffer that causes elution of the nucleic acid having a lower molecular weight than the genomic nucleic acid from the solid phase carriers,
thereby separating genomic nucleic acid of the cell from nucleic acid having a molecular weight that is lower than the molecular weight of the genomic nucleic acid in the cell.

2. A method of separating genomic nucleic acid of a cell from nucleic acid having a molecular weight that is lower than the molecular weight of the genomic nucleic acid in the cell comprising:
a) combining
i) solid phase carriers whose surfaces have bound thereto a functional group which reversibly binds nucleic acid,
ii) a cell lysate and
iii) a reagent, wherein the reagent comprises a nucleic acid precipitation agent which causes precipitation of the nucleic acid of the cell lysate onto the solid phase carriers;
thereby producing a combination;
b) maintaining the combination under conditions in which the nucleic acid of the cell lysate binds reversibly to the solid phase carriers, thereby producing solid phase carriers having nucleic acid of the cell bound thereto;
c) separating the solid phase carriers from the combination;
d) contacting the solid phase carriers with an elution buffer that causes elution of the nucleic acid having a lower molecular weight than the genomic nucleic acid from the solid phase carriers,
thereby separating genomic nucleic acid of the cell from nucleic acid having a molecular weight that is lower than the molecular weight of the genomic nucleic acid in the cell.

3. The method of Claim 1 or Claim 2 wherein the nucleic acid having a lower molecular weight is selected from the group consisting of: plasmid nucleic acid, episomal nucleic acid, mitochondrial nucleic acid, organelle nucleic acid, and viral nucleic acid.

4. The method of Claim 1 or Claim 2 wherein the solid phase carriers comprise paramagnetic microparticles.

5. The method of Claim 1 or Claim 2 wherein the nucleic acid having a lower molecular weight is plasmid nucleic acid and the solid phase carriers comprise paramagnetic microparticles.

6. The method of Claim 4 or Claim 5 wherein the paramagnetic microparticles have a coated surface wherein the coated surface is selected from the group consisting of: a carboxyl group coated surface, an amine group coated surface, and an encapsulated carboxyl group coated surface.

7. The method of Claim 1 or Claim 2 wherein the reagent comprises an alcohol.

8. The method of Claim 7 wherein the alcohol is selected from the group consisting of: ethanol, isopropanol and polyalkylene glycol.

9. The method of Claim 7 wherein the reagent further comprises a salt.

10. The method of Claim 9 wherein the salt is selected from the group consisting of: NaCl, LiCl and MaCl₂.

11. The method of Claim 1 or Claim 2 wherein elution buffer comprises water.

12. The method of Claim 11 wherein the elution buffer further comprises RNAse.

13. The method of Claim 1 or Claim 2 wherein the solid phase carriers are separated from the combination using a method selected from the group consisting of: applying a magnetic field, applying vacuum filtration and applying centrifugation.

14. The method of Claim 2 wherein the cell is selected from the group consisting of: a bacterial cell, a viral nucleic acid containing cell and a mammalian cell.

15. The method of Claim 14 wherein the mammalian cell is a whole blood cell.

## Patentansprüche

1. Verfahren zur Separation einer genomischen Nukleinsäure einer Zelle von einer Nukleinsäure, die ein Molekulargewicht aufweist, das niedriger als das Molekulargewicht der genomischen Nukleinsäure in der Zelle ist, wobei das Verfahren aufweist:
a) ein Kombinieren von
i) Trägern einer festen Phase, deren Oberflächen Bindungen an eine funktionale Gruppe aufweisen, die reversibel die Nukleinsäure bindet,
ii) einer Zelle und
iii) einem Reagenz, wobei das Reagenz einen Lysenbestandteil, der eine Lysis der Zelle bewirkt, und einen Wirkstoff zur Ausfällung von Nukleinsäuren aufweist, der die Ausfällung der Nukleinsäure der Zelle auf die Träger der festen Phase bewirkt,
wodurch eine Zusammensetzung erzeugt wird,
b) ein Aufrechterhalten der Zusammensetzung unter den Bedingungen, bei denen die Lysis der Zelle auftritt und die Nukleinsäure der Zelle reversibel mit den Trägern der festen Phase bindet, wodurch die Träger der festen Phase erzeugt werden, die die Nukleinsäure der Zelle aufweisen; die daran gebunden ist,
c) ein Separieren der Träger der festen Phase aus der Zusammensetzung, und
d) ein Kontaktieren der Träger der festen Phase mit einem Puffer zum Auswaschen, der das Auswaschen der Nukleinsäure aus den Trägern der festen Phase bewirkt, die ein geringeres Molekulargewicht als die genomische Nukleinsäure aufweist,
wodurch die genomische Nukleinsäure der Zelle von der Nukleinsäure separiert wird, die ein Molekulargewicht aufweist, das geringer als das Molekulargewicht der genomischen Nukleinsäure in der Zelle ist.

2. Verfahren zur Separation einer genomischen Nukleinsäure einer Zelle von einer Nukleinsäure, die ein Molekulargewicht aufweist, das niedriger als das Molekulargewicht der genomischen Nukleinsäure in der Zelle ist, wobei das Verfahren aufweist:
a) ein Kombinieren von
i) Trägern einer festen Phase, deren Oberflächen Bindungen an eine funktionale Gruppe aufweisen, die reversibel die Nukleinsäure bindet,
ii) einem Zellenlysaten und
iii) einem Reagenz, wobei das Reagenz einen Wirkstoff zur Ausfällung von Nukleinsäuren aufweist; der die Ausfällung der Nukleinsäure des Zellenlysaten auf die Trägerder festen Phase bewirkt,
wodurch eine Zusammensetzung erzeugt wird,
b) ein Aufrechterhalten der Zusammensetzung unter den Bedingungen, bei denen der Zellenlysat reversibel mit den Trägern der festen Phase bindet, wodurch die Träger der festen Phase erzeugt werden, die die Nukleinsäure der Zelle aufweisen, die daran gebunden ist,
c) ein Separieren der Träger der festen Phase aus der Zusammensetzung, und
d) ein Kontaktieren der Träger der festen Phase mit einem Puffer zum Auswaschen, der das Auswaschen der Nukleinsäure aus den Trägern der festen Phase bewirkt, die ein geringeres Molekulargewicht als die genomische Nukleinsäure aufweist,
wodurch die genomische Nukleinsäure der Zelle von der Nukleinsäure separiert wird, die ein Molekulargewicht aufweist; das geringer als das Molekulargewicht der genomischen Nukleinsäure in der Zelle ist.

3. Verfahren nach Anspruch 1 oder 2, wobei die Nukleinsäure, die ein geringeres Molekulargewicht aufweist, aus der Gruppe ausgewählt worden ist, die umfasst: eine Nukleinsäure eines Plasmids, eine episomale Nukleinsäure, eine mitochondriale Nukleinsäure, eine Nukleinsäure einer Organelle und eine virale Nukleinsäure.

4. Verfahren nach Anspruch 1 oder 2, wobei die Träger der festen Phase paramagnetische Mikropartikel umfassen.

5. Verfahren nach Anspruch 1 oder 2, wobei die Nukleinsäure mit dem geringeren Molekulargewicht eine Nukleinsäure eines Plasmids umfasst und die Träger der festen Phase paramagnetische Mikropartikel umfassen.

6. Verfahren nach Anspruch 4 oder 5, wobei die paramagnetische Mikropartikel eine beschichtete Oberfläche aufweisen, wobei die beschichtete Oberfläche aus einer Gruppe ausgewählt worden ist, die umfasst: eine mit einer Karboxylgruppe beschichteten Oberfläche, eine mit einer Amingruppe beschichteten Oberfläche und eine mit einer eingekapselten Karboxylgruppe beschichteten Oberfläche.

7. Verfahren nach Anspruch 1 oder 2, wobei das Reagenz einen Alkohol aufweist.

8. Verfahren nach Anspruch 7, wobei das Alkohol aus einer Gruppe ausgewählt worden ist, die umfasst: ein Ethanol, ein Isopropanol und ein Polyglykol.

9. Verfahren nach Anspruch 7, wobei das Reagenz ferner ein Salz aufweist.

10. Verfahren nach Anspruch 9, wobei das Salz aus einer Gruppe ausgewählt worden ist, die umfasst: ein NaCl, ein LiCl und ein MgCl₂.

11. Verfahren nach Anspruch 1 oder 2, wobei der Puffer zum Auswaschen Wasser aufweist.

12. Verfahren nach Anspruch 11, wobei der Puffer zum Auswaschen ferner RNAse aufweist.

13. Verfahren nach Anspruch 1 oder 2, wobei die Träger der festen Phase mit einem Verfahren separiert werden, das aus einer Gruppe ausgewählt worden ist, die umfasst: ein Anlegen eines magnetischen Feldes, ein Verwenden einer Vakuumfiltration und ein Verwenden einer Zentrifugierung.

14. Verfahren nach Anspruch 2, wobei die Zelle aus einer Gruppe ausgewählt worden ist, die umfasst: eine bakterielle Zelle, eine eine virale Nukleinsäure enthaltende Zelle und eine Säugetierzelle.

15. Verfahren nach Anspruch 14, wobei die Säugetierzelle eine vollständige Blutzelle ist.

## Revendications

1. Procédé de séparation de l'acide nucléique génomique d'une cellule d'un acide nucléique ayant un poids moléculaire qui est inférieur au poids moléculaire de l'acide nucléique génomique dans la cellule comprenant :
a) la combinaison
i) de supports de phase solide dont les surfaces y ont lié un groupe fonctionnel qui lie de manière réversible de l'acide nucléique,
ii) d'une cellule et
iii) d'un réactif, où le réactif comprend un composant de lyse qui provoque la lyse de la cellule et un agent de précipitation d'acide nucléique qui provoque la précipitation de l'acide nucléique de la cellule sur les supports de phase solide ;
produisant de cette manière une combinaison ;
b) le maintien de la combinaison dans des conditions dans lesquelles la lyse de la cellule se produit et l'acide nucléique de la cellule se lie de manière réversible aux supports de phase solide, produisant de cette manière des supports de phase solide ayant l'acide nucléique de la cellule qui y est lié ;
c) la séparation des supports de phase solide à partir de la combinaison ;
d) la mise en contact des supports de phase solide avec un tampon d'élution qui provoque l'élution de l'acide nucléique ayant un poids moléculaire inférieur à l'acide nucléique génomique depuis les supports de phase solide,
séparant de cette manière l'acide nucléique génomique de la cellule de l'acide nucléique ayant un poids moléculaire qui est inférieur au poids moléculaire de l'acide nucléique génomique dans la cellule.

2. Procédé de séparation de l'acide nucléique génomique d'une cellule d'un acide nucléique ayant un poids moléculaire qui est inférieur au poids moléculaire de l'acide nucléique génomique dans la cellule comprenant :
a) la combinaison
i) de supports de phase solide dont les surfaces y ont lié un groupe fonctionnel qui lie de manière réversible l'acide nucléique,
ii) d'un lysat cellulaire et
iii) d'un réactif, où le réactif comprend un agent de précipitation d'acide nucléique qui provoque la précipitation de l'acide nucléique du lysat cellulaire sur les supports de phase solide ;
produisant de cette manière une combinaison ;
b) le maintien de la combinaison dans des conditions dans lesquelles l'acide nucléique du lysat cellulaire se lie de manière réversible aux supports de phase solide, produisant de cette manière des supports de phase solide ayant l'acide nucléique de la cellule qui y est lié ;
c) la séparation des supports de phase solide à partir de la combinaison ;
d) la mise en contact des supports de phase solide avec un tampon d'élution qui provoque l'élution de l'acide nucléique ayant un poids moléculaire inférieur à l'acide nucléique génomique provenant des supports de phase solide,
séparant de cette manière l'acide nucléique génomique de la cellule de l'acide nucléique ayant un poids moléculaire qui est inférieur au poids moléculaire de l'acide nucléique génomique dans la cellule.

3. Procédé selon la revendication 1 ou la revendication 2, dans lequel l'acide nucléique ayant un poids moléculaire inférieur est choisi dans le groupe constitué : d'acide nucléique plasmidique, d'acide nucléique épisomique, d'acide nucléique mitochondrial, d'acide nucléique d'organite et d'acide nucléique viral.

4. Procédé selon la revendication 1 ou la revendication 2, dans lequel les supports de phase solide comprennent des microparticules paramagnétiques.

5. Procédé selon la revendication 1 ou la revendication 2, dans lequel l'acide nucléique ayant un poids moléculaire inférieur est de l'acide nucléique plasmidique et les supports de phase solide comprennent des microparticules paramagnétiques.

6. Procédé selon la revendication 4 ou la revendication 5, dans lequel les microparticules paramagnétiques ont une surface enrobée où la surface enrobée est choisie dans le groupe constitué : d'une surface enrobée d'un groupe carboxyle, d'une surface enrobée d'un groupe amine et d'une surface enrobée d'un groupe carboxyle encapsulé.

7. Procédé selon la revendication 1 ou la revendication 2, dans lequel le réactif comprend un alcool.

8. Procédé selon la revendication 7, dans lequel l'alcool est choisi dans le groupe constitué : d'éthanol, d'isopropanol et de polyalkylène glycol.

9. Procédé selon la revendication 7, dans lequel le réactif comprend en outre un sel.

10. Procédé selon la revendication 9, dans lequel le sel est choisi dans le groupe constitué de : NaCl, LiCl et MgCl₂.

11. Procédé selon la revendication 1 ou la revendication 2, dans lequel le tampon d'élution comprend de l'eau.

12. Procédé selon la revendication 11, dans lequel le tampon d'élution comprend en outre une ARNase.

13. Procédé selon la revendication 1 ou la revendication 2, dans lequel les supports de phase solide sont séparés de la combinaison en utilisant un procédé choisi dans le groupe constitué : de l'application d'un champ magnétique, de l'application d'une filtration sous vide et de l'application d'une centrifugation.

14. Procédé selon la
revendication 2, dans lequel la cellule est choisie dans le groupe constitué : d'une cellule bactérienne, d'une cellule contenant de l'acide nucléique viral et d'une cellule mammalienne.

15. Procédé selon la revendication 14, dans lequel la cellule mammalienne est une cellule sanguine entière.
